(19) Europäisches Patentamt
European Patent Office
Office européen des brevets

(11) **EP 3 238 645 A1**

(12) **EUROPEAN PATENT APPLICATION**

(43) Date of publication:
**01.11.2017 Bulletin 2017/44**

(51) Int Cl.:
***A61B 18/14*** *(2006.01)*      *A61B 18/00* *(2006.01)*

(21) Application number: **17166387.5**

(22) Date of filing: **12.04.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **15.04.2016 US 201662322861 P**

(71) Applicant: **Cook Medical Technologies LLC Bloomington, IN 47404 (US)**

(72) Inventors:
• **ELGAARD, Per**
  **4690 Haslev (DK)**
• **PAAMAND, Rune Tore**
  **2700 Broenshoej (DK)**
• **TORP, Allan**
  **4632 Bjaeverskov (DK)**
• **BUTZBACKER, Raimo Urban**
  **4690 Haslev (DK)**

(74) Representative: **Garratt, Peter Douglas et al Mathys & Squire LLP The Shard 32 London Bridge Street London SE1 9SG (GB)**

(54) **ABLATION MEDICAL DEVICE WITH BASKET**

(57)     A device to transmit energy to a body vessel has an elongate member; a basket comprising a hub and a circumferential body, the hub disposed about the elongate member, the circumferential body extending radially and distally from the hub to an open end, in an expanded state, the circumferential body having a surface such that a membrane is disposed on the surface, the membrane being substantially impermeable to blood flow such that it occludes the blood flow in the expanded state; a second conductive element being one of the basket and a return electrode disposed outside of the body vessel when the basket is in the expanded state; and a control unit which actuates the device to transmit energy to the body vessel.

FIG. 1

EP 3 238 645 A1

**Description**

FIELD

The present disclosure relates generally to medical devices. More specifically, the disclosure relates to devices and methods for occluding or closing a body vessel using a radiofrequency signal to transmit energy, heat, and/or ablate the body vessel.

BACKGROUND

There are numerous medical conditions when it is desired or necessary to close a body vessel, including the treatment of aneurysms, arteriovenous malformations, arteriovenous fistulas, for starving organs of oxygen and nutrients, in the treatment or containment of cancerous growths, and so on.

Several techniques are known and in use for closing or occluding such body vessels. Traditionally, vessels have been closed by means of external ligation, which generally must be carried out by an open surgery procedure, with its associated risks, inconvenience, and long patient recovery times. Other, more recent, methods aim to use an endoluminal procedure to insert into the vessel or organ one or more occlusion devices, such as a metal framed occluder, coils, pellets or the like, able to obstruct the flow of blood in the vessel.

It is also known to constrict a vessel by endoluminal ablation, causing contraction of the vessel and/or coagulation of blood to form a blood clot in the vessel. Various methods can be employed to cause such ablation.

BRIEF SUMMARY

The invention may include any of the following embodiments in various combinations and may also include any other aspect described below in the written description or in the attached drawings. This disclosure provides a medical device and methods for conducting vessel ablation and occlusion.

The medical device(s) of this disclosure transmits energy to the body vessel to cause vessel ablation. The energy source can be a radiofrequency ("RF") signal generated by a power supply and/or signal generator. The device includes an elongate member including a first conductive element (e.g. first electrode in AC system or anode in DC system) and a basket. The elongate member has a proximal end and extends to a distal end, defining a longitudinal axis A. The elongate member includes the first conducive element.

In one aspect, the basket includes a hub (e.g. first hub) and a circumferential body, the hub disposed about the elongate member. The circumferential body extends radially and distally from the hub to an open end, in an expanded state. The circumferential body has a surface (e.g. inner or outer surface) such that a membrane is disposed on the surface and is substantially impermeable or impeding to blood flow such that the membrane occludes the blood flow in the expanded state.

The device further includes a second conductive element (e.g. second electrode in AC system or cathode in DC system) being one of the basket and a return electrode disposed outside of the body vessel when the circumferential body and device is in the expanded state.

In a second aspect, the basket includes a pair of hubs (e.g. proximal and distal hubs) and a cylindrical portion. The basket is disposed about the elongate member. The cylindrical portion is disposed between the proximal and distal hubs. The cylindrical portion has a first end attached to the proximal hub and distally extends to a second end attached to the distal hub. The cylindrical portion is radially expanded in an expanded state. The cylindrical portion also includes a plurality of struts being interlocked or braided together and having an interlocked or braided structure being substantially impermeable or impeding to the blood flow such that the interlocked structure occludes the blood flow in the expanded state.

In any aspect, the basket (e.g. circumferential body or cylindrical portion) is movable between a collapsed state for delivery and the expanded state for treatment in the vessel. The device further includes a second conductive element being one of the basket, in either aspect, and a return electrode disposed outside of the body vessel in the expanded state.

In any aspect, the device further includes a control unit being operatively coupled to the first conductive element. In the first aspect, the control unit and the first and second conductive elements form a first electrical circuit such that the control unit actuates the device to transmit energy to the body vessel. In the second aspect, the control unit and the first and second conductive elements form a second electrical circuit such that the control unit actuates the device to transmit energy to the body vessel.

In any aspect, the device can be part of an assembly including an outer sheath. The outer sheath has a first sheath end and extending to a second sheath end and forming a lumen therethrough. The elongate member is slidably received within the lumen, having any of the features described herein. The basket in any aspect discussed herein may be disposed about the elongate member in the assembly.

Various additional features and embodiments will become apparent with the following description. The present disclosure may be better understood by referencing the accompanying figures.

BRIEF DESCRIPTION OF THE DRAWINGS

Fig. 1 depicts a partial, environmental view of a medical device in accordance with one aspect of the present disclosure;
Fig. 2 depicts a side view of the device of Fig. 1 in an expanded state;
Fig. 3 depicts a partial, side view of the device of Fig. 1 in a collapsed state;
Fig. 4A depicts a partial, side view of a medical device in accordance with another aspect of the present disclosure;
Fig. 4B depicts a partial, side view of the device of Fig. 4A in an expanded state;
Fig. 5 depicts a partial, side view of the device of Fig. 4A in a collapsed state;
Fig. 6 depicts steps of a method of use of the device of Fig. 1 in accordance with one aspect of the present disclosure; and
Fig. 7 depicts steps of a method of use of the device of Fig. 4B in accordance with another aspect of the present disclosure.

DETAILED DESCRIPTION

The present disclosure will now be described more fully with reference to the accompanying figures, which show various embodiments. The accompanying figures are provided for general understanding of various embodiments and method steps. However, this disclosure may be embodied in many different forms. These figures should not be construed as limiting, and they are not necessarily to scale.

The following definitions will be used in this application.

"About" or "substantially" mean that a given quantity (e.g. lengths or volumes) is within 10%, preferably within 5%, more preferably within 1 % of a stated value. For example, a first quantity of length can be within 10% of a second length quantity. "Substantially impermeable" means that a structure (e.g. basket) blocks essentially all blood flow, while it may have some small pores or holes that allow some substances to pass through and/or a negligable amount of blood flow.

"Adjacent" referred to herein is near, near to, or in close proximity with.

"Longitudinally" and derivatives thereof will be understood to mean along the longitudinal axis of the device.

The terms "proximal" and "distal" and derivatives thereof will be understood in the frame of reference of a physician using the device. Thus, proximal refers to locations closer to the physician and distal refers to the locations farther away from the physician (e.g., deeper in the patient's vasculature).

"Radially" and derivatives thereof will be understood to mean along a radial axis of the body vessel. Likewise, "radial force" and derivatives thereof will be understood to mean a force applied along the radial axis.

The present disclosure discusses a device 10 to perform vessel ablation by transmitting energy to a body vessel having a blood flow. The target vessels for the present disclosure may be any vessel. For example, the target vessels may be small arteries (possibly down to 1 millimeter in diameter) that require a device with a low profile. Generally, the device will have two conductive elements operable to create an electric field. In one example, applying a radiofrequency signal generates the field, and the field heats the local environment in the body vessel (including the blood flow and the vessel wall) to cause swelling and occluding of the body vessel at a target site.

Generally using radiofrequency ("RF") signal, electrical terminal(s)/conductive element(s) are fed endoluminally into the vessel and an electrical pulse and/or constant electrical signal at RF frequencies is applied to the conductive element(s). The conductivity of blood and/or the vessel tissues causes localized heating of the blood and tissue and not significant heating of the conductive elements themselves, creating a local zone or environment that is heated by the RF frequencies. This heating can be used to cause damage to the tissue (intima) of the vessel wall, resulting in vessel contraction. Additionally or alternatively, RF ablation heats the surrounding blood, causing the blood and vessel wall to coagulate around the electrical terminal and form a blood clot and/or embolization which blocks the vessel.

Two types of RF ablation systems are generally contemplated in the art: a monopolar system and a bipolar system. A monopolar system may have an elongate first electrode (e.g. active electrode) and a second electrode (e.g. dispersive electrode) pad or return electrode positioned outside the patient's body. The first electrode is designed to be fed endoluminally into the patient's vessel, while the second electrode pad is positioned against the person's outer body, as close as practicable to the first electrode. In a bipolar system, both electrodes are present in one device and inserted endoluminally into the vessel. Electrical energy applied to the first electrode will pass by conduction to the second electrode. There can be localized heating at the electrodes, which effects the desired ablation.

Advantageously, operating the device having a monopolar system may be easier for manufacturing and use with multiple and/or different return electrode configurations. Alternatively advantageously, operating the device having a bipolar system may be easier for the user by not having a separable return electrode disposable outside of the body.

The RF frequencies, and the power they generate, may not be large enough to heat the first electrode and/or the second or return electrode. However, the RF signal and field generated will cause localized heating of the surrounding blood and tissue. The temperature of the electrodes will likely elevate over body temperature; however, heat is primarily generated in the surrounding tissue.

In one aspect, the RF energy could be an AC signal from about 100kHz to about 1MHz. More specifically, the AC signal could be about 400 to about 500kHz. The signal may also be a DC signal. The signal may vary over time, and can depend on the required power to maintain a desired temperature in the body vessel.

$$\text{Power} = \text{Current} \times \text{Voltage} \qquad \text{Voltage} = \text{Current} \times \text{Resistivity}$$

$$\text{Power} = \text{Current}^2 \times \text{Resistivity} = \text{Voltage}^2/\text{Resistivity}$$

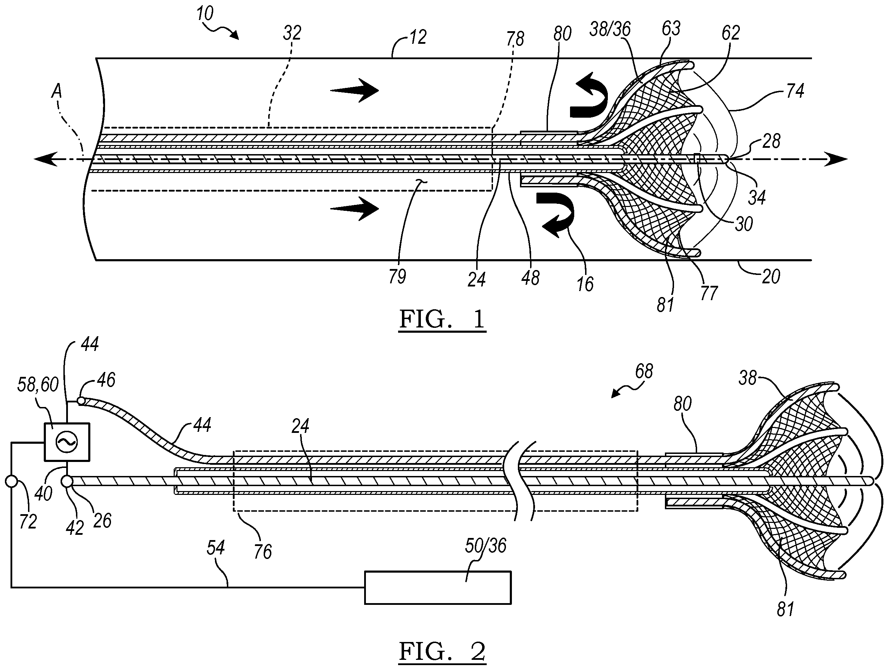

Fig. 1 depicts an environmental view of one aspect of the medical device 10 that may be used to ablate the body vessel 12. The device 10 may include an elongate member 24 including a proximal end (Fig. 2 (26)) and extending to a distal end 28, defining a longitudinal axis A. The elongate member 24 comprises a first conductive element 34. The elongate member 24 may be a modified wire guide with distal end 28. The distal end may include the first conductive element 34.

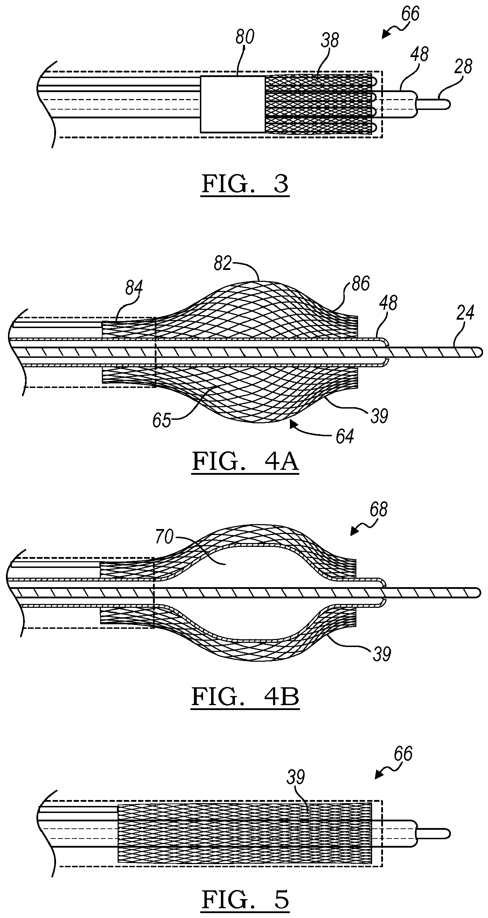

Generally, the device 10 (as depicted in both Figs. 1 and 4A) includes a basket. The basket can be disposed about the elongate member 24. Such baskets can provide blood flow control during vessel ablation that may not be possible with other devices. For example, the basket expands and prevents or impedes blood from flowing in the vessel during the use of the device. Blood flow has a disadvantageous heat sinking effect that any energy delivered to the local environment by the device may be carried downstream by the blood flow. This downstream heating may even have unintended consequences in various sites. However, the baskets of this disclosure can impede, stop, or occlude blood flow until after the procedure to prevent or reduce these unintended consequences.

Controlling or preventing local blood flow can also have an advantage of allowing a practitioner using a device of this disclosure to use lower energy and perform faster embolization and ablation than with devices that do not prevent or reduce local blood flow.

In some cases, the basket itself is a second conductive element for the device (e.g. bipolar system). In general, baskets such as those depicted in this disclosure can provide the advantage of creating a larger electrical field for larger body vessels. Their larger size and surface area can spread out the electrical field. Additionally, these baskets can focus the electrical field between the first and second conductive elements, and assist in centering the first conductive element in the vessel. These, and further, advantages will be discussed in detail below.

In Fig. 1, basket 38 includes a first hub 80 and a circumferential body 81. The first hub 80 is disposed about the elongate member 24. The circumferential body 81 extends radially and distally from the hub 80 to an open end 77. Fig. 1 depicts the circumferential body and device an expanded state, having the open end 77 contacting the vessel wall 20. The circumferential body 81 also has an outer surface such that a membrane 62 is disposed on the outer surface. The membrane 62 is substantially impermeable to the blood flow 16 such that the membrane 62 occludes a blood flow 16 in the expanded state. As discussed herein, the membrane 62 occluding the blood flow 16 provides the advantage of reducing or preventing the heat sinking effect of the blood flow during ablation. Minimal heat delivered to the vessel will be lost downstream due to blood flow. This allows the user to have greater control over ablation. The membrane 62 can be formed in any manner known in the art, including the membrane being a coating on the outer surface. The membrane could also be applied on an inner surface of the basket 38.

Also as discussed above, the device includes a second conductive element 36 being one of the basket 38 and a return electrode (Fig. 2 (50)) disposed outside of the body vessel 12 when the circumferential body and device is in the expanded state. As will be understood, if the basket 38 functions as the second conductive element, the device operates as a bipolar system. Alternatively, if the return electrode or return pad disposed outside of the body functions as the second conductive element, the device operates as a monopolar system. It is also possible that one device could have both the basket 38 and the return electrode capable of functioning as the second conductive element, and the user may determine which to use for a given procedure.

The device 10 also includes a control unit (Fig. 2 (58)) operatively coupled to the first and second conductive elements (34, 36). The control unit and first and second conductive elements form a first electrical circuit such that the control unit actuates the device to transmit energy to the body vessel 12. Optionally with a DC system, the first conductive element 34 is an anode and the second conductive element 36 is a cathode. Alternatively with an AC system, the first and second conductive elements are electrodes. Fig. 1 depicts the electrical field 74 generated by the control unit. It will be understood that the schematic drawing of electrical field 74 is merely illustrative of an electrical field between two electrodes, and the electrons within this field are not necessarily located in only the locations of these schematic lines.

The device may optionally further include an outer sheath 32 having a first sheath end (Fig. 2 (76)) adjacent the proximal end and a second sheath end 78 proximal and adjacent the distal end 28. The outer sheath 32 is shown as a dotted line, and the internal components of the device are shown in solid lines. However, one of skill in this art will understand that the internal components would normally be obscured by the outer sheath 32. Similarly, the hub 80 is illustrated in a cross-sectional view such that the internal components (e.g. elongate member 24) are visible, which would normally be obscured by the hub 80.

The outer sheath 32 forms a lumen 79 therethrough such that the elongate member 24 is slidably disposed in the lumen 79. If the second conductive element 36 is the basket 38, the control unit and the first and second conductive elements form the first electrical circuit. In this case, the second conductive element 36 is circumferentially disposed about the longitudinal axis and the elongate member 24.

Alternatively, if the second conductive element 36 is the return electrode, the control unit, the first conductive element, and the return electrode form another type of electrical circuit having a modified path. Additionally or alternatively, the elongate member 24 may form a loop or curved hook at its respective distal end so that it comes into closer contact with the vessel wall during use. As the vessel wall swells, the curved distal end may come into contact with the vessel wall and facilitate ablation and/or heating. For example, the first elongate member 24 can have a loop or hook at the distal end 28.

The device 10 can further include an isolator 48 disposed about the first conductive element 34 to electrically isolate and/or insulate the first conductive element 34 from the rest of the device. The isolator 48 could be a shrink tubing heat shrunk and/or immoblized about or onto the first elongate member 24. The isolator 48 may have a first isolator end distal the proximal end 26. The isolator may extend to a second isolator end proximal the distal end 28, creating exposed ends of the elongate member. In this way, even if the first conductive element 34 extends from the proximal end 26 and to the distal end 28, it will have a central region electrically isolated from the second conductive element 36 and the rest of the device.

The first hub 80 may be fixedly attached to the isolator 48 such that the first hub 80 and the elongate member do not move relative to each other. However, the first hub 80 could also be slidably disposed about the isolator 48, including the isolator 48 having a lubricious coating to accommodate easy sliding between components.

Additionally, the device 10 can include a temperature sensor 30 (thermistor, thermocouple, and the like) integrated into the device at a suitable location. In Fig. 1, the temperature sensor 30 is located adjacent the distal end 28. The temperature sensor could also be at the distal end 28. In this aspect, the temperature sensor 30 could act as both a temperature sensor and the first conductive element/electrode 34. In a second aspect, the temperature sensor 30 could be electrically isolated from the first conductive element 34, but physically integrated with it (e.g. mounted on the isolator 48) adjacent and/or proximal to the distal end 28.

In a third aspect, the temperature sensor could be integrated at one end (e.g. 78) of an outer sheath 32 (e.g. active or passive microcatheter). In a fourth aspect, the temperature sensor could be a separate device mounted at the tip of a delivery wire, allowing completely independent positioning of the temperature sensor relative to the conductive elements and other parts of the device.

In any case, sufficient electrical shielding may be needed between components of the device 10 conducting RF energy and the temperature sensor because it can be difficult to sense temperature and conduct energy/electricity reliably if these components are too close to each other. One way of overcoming this interference is to alternate between ablation and temperature measurement so that ablation is performed out-of-phase with the temperature measurement. In one aspect, the practitioner may ablate for 900 milliseconds, and then measured temperature for 100 milliseconds, continuously repeating this 1 second pattern. Other time increments are also contemplated.

Another possible solution to the possible interference between temperature sensing and conducting energy is by electrical filtering of the temperature signal. Some examples include using RF chokes to block higher-frequency AC and/or low-pass filtering where the high-frequency interference is attenuated and the lower-frequency temperature signal is admitted.

It is also contemplated to use a first material for the temperature sensor 30 and a different, second material for the conductive elements (34, 36) to further avoid any interference. In one example, the temperature sensor and/or thermistor could be made of Nifethal® (a nickel/iron alloy), available from Kanthal, Hallstahmmar, Sweden. Temperature could be measured by the change in resistance of the material used.

Fig. 2 depicts a side view of the device of Fig. 1, also including the proximal end with the control unit 58. The control unit 58 may also include a signal generator 60 to generate the RF signal. Additionally, the electrical circuits of this disclosure can include other components or parts as needed and/or desired for form the circuit(s). For example, a first connector or wire 40 can optionally be connected and form a junction 42 between the control unit 58 and the elongate member 24 and/or first conductive element 34. A second connector or wire 44 can optionally be connected to the control unit 58 and the basket 38. The second wire 44 may include a junction 46. Likewise, a third connector or wire 54 may extend from the control unit 58 to the return electrode 50 or pad, having another junction 72.

In one example, the second connector 44 is connected to the control unit 58 and extends to the basket 38. The elongate member 24 can be directly connected to the control unit 58 or connected to the control unit 58 by way of the first connector 40. In one example, the control unit 58, the elongate member 24, the basket 38, and the second connector 44 form the first electrical circuit. The first connector 40 may also be included in the first electrical circuit.

While Fig. 2 depicts field lines similar to Fig. 1, it will be understood that if the basket 38 functions as the second conductive element, these field lines would be located between the basket 38 and the first conductive element. In this example, it is also possible that the device could include a return electrode 50, which could also function as the second conductive element if the user desired to use the device in a monopolar mode. One device could be used in two different modes. Further, in a device where the basket 38 did not include the second conductive element, the field lines shown in Fig. 2 would not be present. Instead, the return electrode or pad 50 would function as the second conductive element, and the electrical field would be generated between the first conductive element and the return electrode 50.

The first hub 80 includes a tubular segment disposed about the elongate member 24. The circumferential body 81 includes a plurality of ribs or struts (e.g. 63) wherein each strut 63 has a first strut end connected to the first hub 80 and extends radially and distally from the tubular segment to the open end 77 in the expanded state. The plurality of struts could have the overall geometry of a spherical cap, polygon, ellipsoid, and the like when the basket is in the expanded state.

While Fig. 2 depicts the basket 38 in an expanded state, Fig. 3 depicts the basket 38 of Fig. 1 in a collapsed state 66. The basket 38 can move between the collapsed and expanded states in a variety of manners known in the art. For example, the basket can be self-expanded such that it automatically expands after withdrawing the outer sheath. Additionally, materials forming the basket 38 can assist in its self-expanding capabilities. For example, Nitinol® is a material with self-expanding properties. The basket could also be made from a material that is soft, flexible, and electrically favorable, such as platinum and/or alloys thereof.

Nitinol is a metal alloy of nickel and titanium having a unique shape memory setting property and being biocompatible. At a transition temperature, Nitinol may undergo a phase change from Martensitic to Austenite, changing its structure. Here, the basket could be heat set or pre-set to the expanded state and maintained in the collapsed or delivery state (shown in Figure 3) until deployed. In addition to this phase changing ability, Nitinol is also quite flexible, having superelastic properties. Additionally, the heat or temperature change which causes the basket 38 to self-expand could be generated by the RF signal or current flowing through the basket 38 during use. Alternatively or additionally, the basket 38 could self-expand by being springloaded or balloon expandable.

Turning to a second aspect, Fig. 4A depicts another way of forming a basket 39. This basket could also be formed of the materials discussed herein, and have similar properties to basket 38. The device of Fig. 4A also includes the elongate member 24 including a proximal end (Fig. 2 (26)) and extending to a distal end, defining a longitudinal axis. The basket 39 includes a proximal hub 84, a distal hub 86, and a cylindrical or tubular portion 82. The proximal hub 84 is disposed about the elongate member 24. The cylindrical portion 82 has a first end attached to the proximal hub 84, and extends distally from the proximal hub 84 to a second end attached to the distal hub 86 (radially tapering), in an expanded state. The proximal hub 84, distal hub 86, and cylindrical portion 82 could be integrally formed (as depicted). The hubs could also be formed by rings and attached to the basket 39 through any means know in the art (e.g. gluing, soldering, welding, and the like) to cause the basket 39 to taper around the elongate member 24. In Fig. 4A, the basket 39 is shown in a cross sectional view such that the internal components (e.g. elongate member 24) are visible. However, the basket 39 would be disposed around the entire circumference of the elongate member 24. Like Fig. 1, the outer sheath is also shown in dotted lines so that internal components are visable.

The proximal hub 84 and the cylindrical portion 82 have one or more struts (e.g. 65) being braided or interlocked together and forming an interlocked or braided structure 64. The interlocked structure 64 is substantially impermeable to the blood flow such that it occludes the blood flow in the expanded state, having the advantages discussed herein. In some examples, the interlocked structure 64 can block or occlude greater than about 50% of the blood flow to about 100% of the blood flow by covering the cross-sectional area of the vessel. In this way, the interlocked structure 64 can impede or be substantially impermeable to the blood flow. Additionally or alternatively, a membrane similar to membrane 62 can be disposed about the surface (e.g. inner or outer) of basket 39 to occlude or assist in occluding blocking of blood flow.

The device of Fig. 4A also includes a second conductive element being one of the basket 39 and a return electrode. The return electrode is disposed outside of the body vessel when the cylindrical portion and device is in the expanded state (as shown in Fig. 2 (50)). The device also includes a control unit being operatively coupled to the first and second conductive elements, forming a second electrical circuit such that the control unit actuates the device to transmit energy to the body vessel. The control unit has all or any of the features discussed above with Fig. 2.

The basket 39 can be formed by any manner known in the art to form a tight weave that blocks or impedes blood flow. For example, the basket 39 can be formed of a plurality of struts being interlocked, woven, knotted, braided, stamped, or cut to form the interlocked structure 64. The interlocked structure 64 can extend or be formed on the entire outer surface of the basket 39.

The features of the outer sheath 32, isolator 48, first and second conductive elements (34, 36), and various circuits discussed above with the first aspect can also apply to this second aspect. For example, the hubs (84, 86) could be fixed or slidable over the isolator 48. Also similar to the first aspect, the second aspect disclosed in Figs. 4A, 4B, and 5 may also be part of an assembly with the outer sheath.

Likewise, Fig. 4B depicts the basket 39 in an expanded state 68. Fig. 5 depicts the basket 39 in its collapsed state 66, within the outer sheath. The basket 39 can expand from the collapsed state to the expanded state in the same ways as discussed with Fig. 3. Additionally shown in Fig. 4B, a balloon 70 may be disposed within the basket 39. The basket 39 is depicted in a cross-sectional view to show the balloon 70. Balloon 70 could be formed as a layer disposed around the isolator and elongate member. The balloon 70 may be expandable and deflatable by a practitioner to move the device from the collapsed state to the expanded state. Balloon 70 can also occlude and/or assist in occluding the blood flow, making the basket 39 substantially impermeable.

It will be appreciated that either basket 38 or 39 could facilitate centering the elongate member 24 and the first conductive element 34 in the body vessel and device. This may have the advantage of creating an electrical field that extends through largest volume possible in the vessel, and this may allow a focused electrical field between the first and second conductive elements.

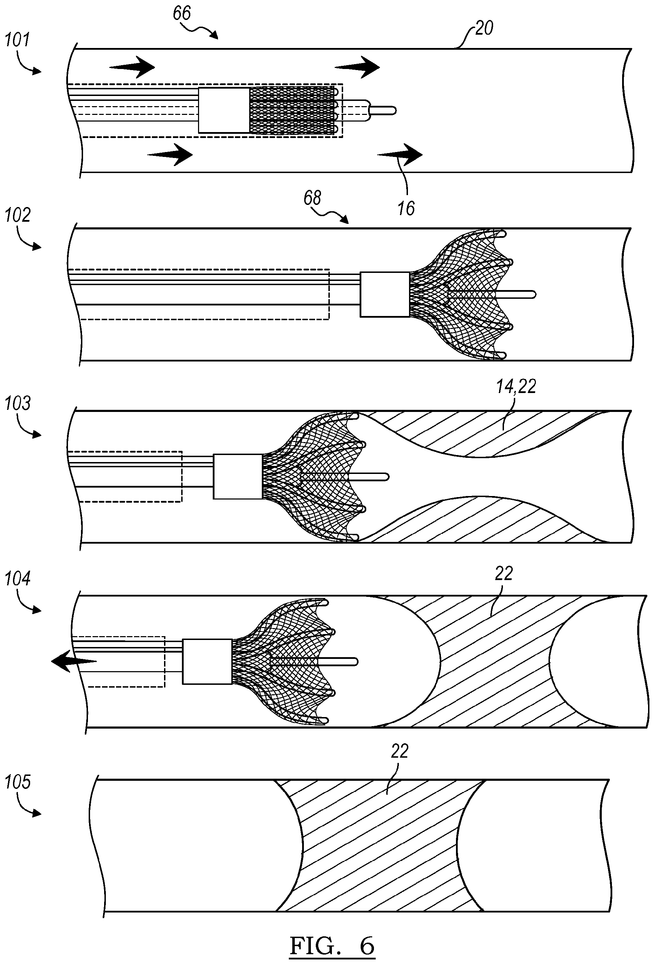

Fig. 6 depicts steps of a method of use of the device of Fig. 1. In step 101, the basket is in the collapsed state 66 within the outer sheath, not occluding blood flow 16. In step 102, the practitioner removes and/or withdraws proximally the outer sheath to expose the basket and allow the basket to expand to its expanded state 68. In step 103, the practitioner operates the control unit to start ablation, forming an occlusion 22 and blood coagulation 14. Embolization and occlusion starts to form. The user may slowly withdraw the device as the occlusion forms.

In step 104, the occlusion 22 has fully formed and the vessel is closed. In step 105, the user or practitioner withdraws the device, leaving the occlusion 22 intact.

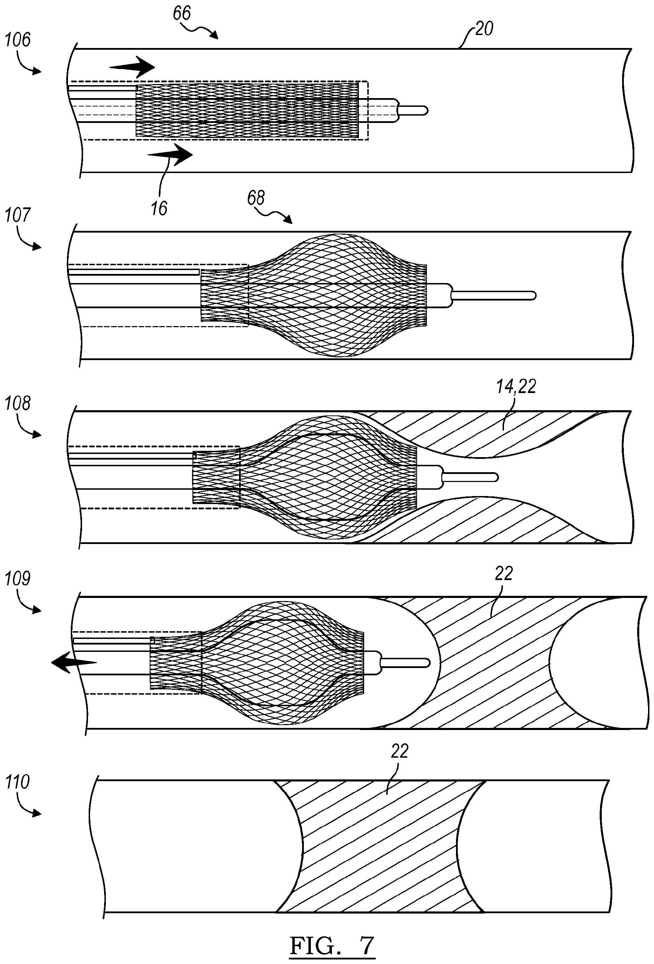

Similarly in Fig. 7, method steps of another method of ablation with a device according to the second aspect are shown. In step 106, the device is delivered with the basket in the collapsed state 66 to a target site having a vessel wall 20. Because the basket is collapsed, it does not include blood flow 16. In step 107, the practitioner withdraws the outer sheath and the basket expands to the expanded state 68, occluding blood flow. Here, a balloon can facilitate expansion. Alternatively, the device may not have a balloon, and a self-expanding structure facilitates expansion.

In step 108, the practitioner operates the device such that the RF signal and field start to form blood coagulation 14 and occlusion 22. The user may slowly withdraw the device as the occlusion forms. In step 109, the occlusion 22 is fully formed distal the basket. In step 110, the practitioner withdraws the device, leaving the occlusion 22 intact. Through these aspects, the inventors demonstrate various devices and methods to perform vessel ablation with an energy source.

FEATURE COMBINATIONS

The following feature combinations assist in understanding this disclosure. The device includes an elongate member including a first conductive element and a basket. The elongate member has a proximal end and extends to a distal end, defining a longitudinal axis A. The elongate member includes the first conducive element. The basket includes a proximal hub and a circumferential body, the proximal hub disposed about the elongate member. The circumferential body extends radially and distally from the proximal hub to an open end, in an expanded state. The circumferential body has a surface such that a membrane is disposed on the surface and is substantially impermeable to blood flow such that the membrane occludes the blood flow in the expanded state. The device further includes a second conductive element being one of the basket and a return electrode disposed outside of the body vessel in the expanded state.

The device includes a control unit being operatively coupled to the first conductive element. In the first aspect, the control unit and the first and second conductive elements form a first electrical circuit such that the control unit actuates the device to transmit energy to the body vessel.

Additionally or alternatively to the above combination(s), the device can optionally be part of an assembly including an outer sheath. The outer sheath has a first sheath end and extending to a second sheath end and forming a lumen therethrough. The elongate member is slidably received within the lumen. The basket may be disposed about the elongate member in the assembly.

Additionally or alternatively to the above combination(s), the distal end can optionally include the first conductive element, and the second conductive element is the basket such that the control unit, the first conductive element, and the second conductive element form the first electrical circuit.

Additionally or alternatively to the above combination(s), the second conductive element is optionally circumferentially disposed about the longitudinal axis.

Additionally or alternatively to the above combination(s), the device can optionally include a first connector connected to the control unit and extending to the basket, the elongate member being connected to the control unit such that the control unit, the elongate member, the basket, and the first connector form the first electrical circuit.

Additionally or alternatively to the above combination(s), the proximal hub can optionally include a tubular segment disposed about the elongate member, and the circumferential body comprises a plurality of ribs, each rib having a first end connected to the tubular segment, each rib extending radially and distally from the hub to the open end in the expanded state.

Additionally or alternatively to the above combination(s), the plurality of ribs can optionally form a spherical cap in the expanded state.

Additionally or alternatively to the above combination(s), the membrane can optionally be formed as a coating, which can be disposed on the entire surface of the basket.

Additionally or alternatively to the above combination(s), the device may optionally include an isolator disposed about the first conductive element to electrically isolate the first conductive element.

Additionally or alternatively to the above combination(s), the device may optionally include a temperature sensor.

Additionally or alternatively to the above combination(s), the second conductive element is the return electrode.

In a second aspect, the device has an elongate member including a first conductive element and a basket. The elongate member has a proximal end and extends to a distal end, defining a longitudinal axis A. The elongate member includes the first conducive element. The basket includes a pair of hubs and a cylindrical portion disposed therebetween. The cylindrical portion has a first end attached to the proximal hub and distally extends to a second end attached to the distal hub. The cylindrical portion is radially expanded in an expanded state, and the cylindrical portion includes a plurality of struts being interlocked together and forming an interlocked structure or braided structure being substantially impermeable or impeding to the blood flow such that the interlocked structure occludes the blood flow in the expanded state. The device further includes a second conductive element being one of the basket and a return electrode disposed outside of the body vessel in the expanded state.

The device includes a control unit being operatively coupled to the first conductive element. In the first aspect, the control unit and the first and second conductive elements form a second electrical circuit such that the control unit actuates the device to transmit energy to the body vessel.

Additionally or alternatively to the above combination(s), the device can optionally be part of an assembly including an outer sheath. The outer sheath has a first sheath end and extending to a second sheath end and forming a lumen therethrough. The elongate member is slidably received within the lumen. The basket may be disposed about the elongate member in the assembly.

Additionally or alternatively to the above combination(s), the distal end can optionally include the first conductive element, and the second conductive element is the basket such that the control unit, the first conductive element, and the second conductive element form the second electrical circuit.

Additionally or alternatively to the above combination(s), the second conductive element is optionally circumferentially disposed about the longitudinal axis.

Additionally or alternatively to the above combination(s), the device can optionally include a first connector connected to the control unit and extending to the basket, the elongate member being connected to the control unit such that the control unit, the elongate member, the basket, and the first connector form the second electrical circuit.

Additionally or alternatively to the above combination(s), the device may optionally include an isolator disposed about the first conductive element to electrically isolate the first conductive element.

Additionally or alternatively to the above combination(s), the device may optionally include a temperature sensor.

Additionally or alternatively to the above combination(s), the second conductive element is the return electrode.

Additionally or alternatively to the above combination(s), the device may optionally include a balloon disposed inside the basket, the balloon being inflatable to move the basket from a collapsed state to the expanded state.

It should be understood that the foregoing relates to exemplary embodiments of the disclosure and that modifications may be made without departing from the spirit and scope of the disclosure as set forth in the following claims. While the disclosure has been described with respect to certain embodiments it will be appreciated that modifications and changes may be made by those skilled in the art without departing from the spirit of the disclosure.

**Claims**

1. A device to transmit energy to a body vessel having a blood flow, the device comprising:

   an elongate member comprising a proximal end and extending to a distal end, defining a longitudinal axis, the elongate member comprising a first conductive element;
   a basket comprising a hub and a circumferential body, the hub disposed about the elongate member, the circumferential body extending radially and distally from the hub to an open end, in an expanded state, the circumferential body having a surface such that a membrane is disposed on the surface, the membrane being substantially impermeable to blood flow such that it occludes the blood flow in the expanded state;

a second conductive element being one of the basket and a return electrode disposed outside of the body vessel when the basket is in the expanded state; and

a control unit being operatively coupled to the first and second conductive elements, forming a first electrical circuit such that the control unit actuates the device to transmit energy to the body vessel.

2. The device of claim 1 further comprising an outer sheath having a first sheath end adjacent the proximal end and a second sheath end adjacent the distal end, the outer sheath extending from the first sheath end to the second sheath end and forming a lumen therethrough, the elongate member being slidably disposed in the lumen.

3. The device of claim 1 or claim 2 wherein the distal end comprises the first conductive element, and the second conductive element is the basket such that the control unit, the first conductive element, and the second conductive element form the first electrical circuit, preferably wherein the second conductive element is circumferentially disposed about the longitudinal axis.

4. The device of claim 3 further comprising a second connector connected to the control unit and extending to the basket, the elongate member being connected to the control unit such that the control unit, the elongate member, the basket, and the first connector form the first electrical circuit.

5. The device of any one of the preceding claims wherein the hub comprises a tubular segment disposed about the elongate member, and the circumferential body comprises a plurality of struts, each strut having a first strut end connected to the tubular segment, each strut extending radially and distally from the hub to the open end in the expanded state, preferably wherein the plurality of struts forms a spherical cap in the expanded state..

6. The device of any one of the preceding claims wherein the membrane is a coating.

7. The device of any one of the preceding claims further comprising an isolator disposed about the first conductive element to electrically isolate the first conductive element.

8. The device of any one of the preceding claims further comprising a temperature sensor.

9. A device to transmit energy to a body vessel having a blood flow, the device comprising:

an elongate member comprising a proximal end and extending to a distal end, defining a longitudinal axis, the elongate member comprising a first conductive element;

a basket disposed about the elongate member and comprising a proximal hub, a distal hub, and a cylindrical portion disposed therebetween, the cylindrical portion having a first end attached to the proximal hub and distally extending to a second end attached to the distal hub, the cylindrical portion being radially expanded in an expanded state, the cylindrical portion comprising a plurality of struts being interlocked together and forming an interlocked structure being substantially impermeable or impeding to the blood flow such that the interlocked structure occludes the blood flow in the expanded state;

a second conductive element being one of the basket and a return electrode disposed outside of the body vessel when the basket is in the expanded state; and

a control unit being operatively coupled to the first and second conductive elements, forming a second electrical circuit such that the control unit actuates the device to transmit energy to the body vessel.

10. The device of claim 9 further comprising an outer sheath having a first sheath end adjacent the proximal end and a second sheath end adjacent the distal end, the outer sheath extending from the first sheath end to the second sheath end and forming a lumen therethrough, the elongate member being slidably disposed in the lumen.

11. The device of claim 9 or claim 10 wherein the distal end comprises the first conductive element, and the second conductive element is the basket such that the control unit, the first conductive element, and the second conductive element forming the second electrical circuit.

12. The device of claim 11 wherein the second conductive element is circumferentially disposed about the longitudinal axis.

13. The device of claim 12 further comprising a first connector connected to the control unit and extending to the basket, the elongate member being connected to the control unit such that the control unit, the elongate member, the basket, and the first connector form the second electrical circuit.

14. The device of any one of claim 9 to claim 13 further comprising an isolator disposed about the first conductive element to electrically isolate the first conductive element.

15. The device of any one of claim 9 to claim 14 further comprising a balloon disposed inside the basket, the balloon being inflatable to move the basket from a collapsed state to the expanded state.

FIG. 1

FIG. 2

FIG. 3

FIG. 4A

FIG. 4B

FIG. 5

FIG. 6

FIG. 7

Europäisches
Patentamt

European
Patent Office

Office européen
des brevets

# EUROPEAN SEARCH REPORT

Application Number

EP 17 16 6387

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/157987 A1 (STEINKE TOM A [US] ET AL) 21 June 2012 (2012-06-21) | 1-6, 8-13,15 | INV. A61B18/14 |
| Y | * paragraphs [0013], [0118], [0119], [0080]; figures 2A,31 * <br> * paragraphs [0063], [0086], [0116], [0083]; figures 3,17,11,2 * | 7,14 | ADD. A61B18/00 |
| X | US 2009/062873 A1 (WU ANDREW [US] ET AL) 5 March 2009 (2009-03-05) * paragraphs [0081], [0082], [0064], [0053]; figures 6A,6B * * paragraph [0085]; figures 7A,7B * | 1,9 | |
| Y | US 6 582 423 B1 (THAPLIYAL HIRA V [US] ET AL) 24 June 2003 (2003-06-24) * figures 2A-2C * | 7,14 | |
| A | US 2015/025532 A1 (HANSON CASS A [US] ET AL) 22 January 2015 (2015-01-22) * figure 11 * | 1-15 | |
| A | US 2012/232326 A1 (HABIB NAGY [GB]) 13 September 2012 (2012-09-13) * figures 9,10 * | 1-15 | TECHNICAL FIELDS SEARCHED (IPC) <br> A61B |

The present search report has been drawn up for all claims

| Place of search | Date of completion of the search | Examiner |
|---|---|---|
| Munich | 15 September 2017 | Monogyiou, Efstratia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 238 645 A1**

## ANNEX TO THE EUROPEAN SEARCH REPORT
## ON EUROPEAN PATENT APPLICATION NO.

EP 17 16 6387

15-09-2017

| Patent document cited in search report | | Publication date | Patent family member(s) | | Publication date |
|---|---|---|---|---|---|
| US 2012157987 | A1 | 21-06-2012 | AU | 2004285412 A1 | 12-05-2005 |
| | | | CA | 2539026 A1 | 12-05-2005 |
| | | | CA | 2938411 A1 | 12-05-2005 |
| | | | CN | 1867299 A | 22-11-2006 |
| | | | DE | 202004021941 U1 | 13-05-2013 |
| | | | DE | 202004021942 U1 | 13-05-2013 |
| | | | DE | 202004021943 U1 | 13-05-2013 |
| | | | DE | 202004021944 U1 | 16-07-2013 |
| | | | DE | 202004021946 U1 | 29-05-2013 |
| | | | DE | 202004021947 U1 | 13-05-2013 |
| | | | DE | 202004021949 U1 | 27-05-2013 |
| | | | DE | 202004021950 U1 | 19-06-2013 |
| | | | DE | 202004021951 U1 | 19-06-2013 |
| | | | DE | 202004021952 U1 | 19-06-2013 |
| | | | DE | 202004021953 U1 | 19-06-2013 |
| | | | DE | 202004021954 U1 | 19-06-2013 |
| | | | EP | 1667595 A2 | 14-06-2006 |
| | | | EP | 2452648 A1 | 16-05-2012 |
| | | | EP | 3045136 A1 | 20-07-2016 |
| | | | ES | 2564694 T3 | 28-03-2016 |
| | | | JP | 2007504910 A | 08-03-2007 |
| | | | US | 2005096647 A1 | 05-05-2005 |
| | | | US | 2008161801 A1 | 03-07-2008 |
| | | | US | 2012157987 A1 | 21-06-2012 |
| | | | US | 2013066316 A1 | 14-03-2013 |
| | | | US | 2013274658 A1 | 17-10-2013 |
| | | | US | 2017056105 A1 | 02-03-2017 |
| | | | WO | 2005041748 A2 | 12-05-2005 |
| US 2009062873 | A1 | 05-03-2009 | AT | 494040 T | 15-01-2011 |
| | | | AT | 536147 T | 15-12-2011 |
| | | | CN | 101610735 A | 23-12-2009 |
| | | | CN | 103222894 A | 31-07-2013 |
| | | | CN | 105056408 A | 18-11-2015 |
| | | | DE | 202007019566 U1 | 31-10-2013 |
| | | | EP | 2037840 A2 | 25-03-2009 |
| | | | EP | 2092957 A1 | 26-08-2009 |
| | | | EP | 2218479 A2 | 18-08-2010 |
| | | | EP | 2465470 A1 | 20-06-2012 |
| | | | EP | 2465574 A1 | 20-06-2012 |
| | | | EP | 2842604 A1 | 04-03-2015 |
| | | | ES | 2361583 T3 | 20-06-2011 |
| | | | ES | 2378956 T3 | 19-04-2012 |
| | | | ES | 2560180 T3 | 17-02-2016 |
| | | | US | 2008255642 A1 | 16-10-2008 |
| | | | US | 2009062873 A1 | 05-03-2009 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 17 16 6387

This annex lists the patent family members relating to the patent documents cited in  the above-mentioned European search report.
The members are as contained in the European Patent Office EDP file on
The European Patent Office is in no way liable for these particulars which are merely given for the purpose of information.

15-09-2017

| Patent document cited in search report | | Publication date | Patent family member(s) | | Publication date |
|---|---|---|---|---|---|
| | | | US | 2009076409 A1 | 19-03-2009 |
| | | | US | 2017014177 A1 | 19-01-2017 |
| | | | WO | 2008003058 A2 | 03-01-2008 |
| US 6582423 | B1 | 24-06-2003 | AU | 7141198 A | 30-12-1998 |
| | | | CA | 2287206 A1 | 17-12-1998 |
| | | | EP | 0998248 A1 | 10-05-2000 |
| | | | JP | 3391466 B2 | 31-03-2003 |
| | | | JP | 2000515798 A | 28-11-2000 |
| | | | US | 6582423 B1 | 24-06-2003 |
| | | | WO | 9856324 A1 | 17-12-1998 |
| US 2015025532 | A1 | 22-01-2015 | CN | 105555220 A | 04-05-2016 |
| | | | EP | 3024406 A1 | 01-06-2016 |
| | | | JP | 2016527959 A | 15-09-2016 |
| | | | US | 2015025532 A1 | 22-01-2015 |
| | | | WO | 2015013205 A1 | 29-01-2015 |
| US 2012232326 | A1 | 13-09-2012 | CN | 102686180 A | 19-09-2012 |
| | | | EP | 2496165 A2 | 12-09-2012 |
| | | | US | 2012232326 A1 | 13-09-2012 |
| | | | WO | 2011055143 A2 | 12-05-2011 |

EPO FORM P0459

For more details about this annex : see Official Journal of the European Patent Office, No. 12/82

page 2 of 2